(19) Europäisches Patentamt
European Patent Office
Office européen des brevets

(11) Veröffentlichungsnummer: **0 352 673 B1**

(12) **EUROPÄISCHE PATENTSCHRIFT**

(45) Veröffentlichungstag der Patentschrift: **09.03.94**

(21) Anmeldenummer: **89113500.6**

(22) Anmeldetag: **22.07.89**

(51) Int. Cl.5: **C07D 405/06**, C07D 405/14, C07D 233/56, C07D 249/08, C07D 233/60, C07D 233/61, A01N 43/50, A01N 43/653

(54) **1-Chlor-1-azolyl-methyloxirane und diese enthaltende Fungizide.**

(30) Priorität: **29.07.88 DE 3825841**

(43) Veröffentlichungstag der Anmeldung:
**31.01.90 Patentblatt 90/05**

(45) Bekanntmachung des Hinweises auf die Patenterteilung:
**09.03.94 Patentblatt 94/10**

(84) Benannte Vertragsstaaten:
**AT BE CH DE ES FR GB GR IT LI NL SE**

(56) Entgegenhaltungen:
**EP-A- 0 004 315** **EP-A- 0 090 269**
**EP-A- 0 094 564** **EP-A- 0 118 245**
**EP-A- 0 132 730** **EP-A- 0 163 606**
**EP-A- 0 196 038** **DE-A- 2 652 313**

(73) Patentinhaber: **BASF Aktiengesellschaft**
**Carl-Bosch-Strasse 38**
**D-67063 Ludwigshafen(DE)**

(72) Erfinder: **Seele, Rainer, Dr.**
**Leiblstrasse 3**
**D-6701 Fussgoenheim(DE)**
Erfinder: **Kober, Reiner, Dr.**
**Im Schlittweg 20**
**D-6701 Fussgoenheim(DE)**
Erfinder: **Goetz, Norbert, Dr.**
**Schoefferstrasse 25**
**D-6520 Worms 1(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die vorliegende Erfindung betrifft neue Azolverbindungen, Verfahren zu ihrer Herstellung und diese enthaltende Fungizide, sowie Verfahren zur Bekämpfung von Pilzen.

Es ist bekannt, Triazolylmethyloxirane, z.B. das 2-(1,2,4-Triazol-1-yl-methyl)-2-phenyl-3-(4-chlorphenyl)-oxiran oder das 2-(Imidazol-1-yl-methyl)-2-phenyl-3-(4-chlorphenyl)-oxiran (DE-32 18 130.2, EP-A-94 564) als Fungizide zu verwenden. Die fungiziden Wirkungen sind jedoch unbefriedigend. Es ist ferner bekannt, Azolylmethyloxirane (EP-A-196 038), 1,3-Ditriazolyl-propan-2-ole (EP-A-118 245), 3-Azolyl-1,2-diaryl-1-halogen-1-propene (EP-A-90 269), 3-(1,2,4-Triazol-1-yl)-1-propene (EP-A-132 730) und 1-Triazol-2,3-diaryl-2-propene (DE-A-2 652 313) als Fungizide zu verwenden.

Es wurde nun gefunden, daß 1-Halogen-1-azolylmethyloxirane der Formel I

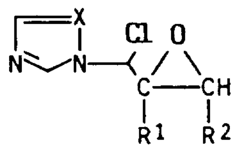

I

in welcher

$R^1$ und $R^2$ gleich oder verschieden sind und $C_1$-$C_8$-Alkyl, $C_5$-$C_8$-Cycloalkyl, $C_5$-$C_8$-Cycloalkenyl, Naphthyl, Biphenyl oder Phenyl bedeuten, wobei diese Reste einfach bis dreifach durch Halogen, Nitro, Phenoxy, Amino, Alkyl, Alkoxy oder Halogenalkyl mit jeweils bis 4 C-Atomen substituiert sein können,

X den Rest CH oder N bedeutet,

sowie deren für Pflanzen verträgliche Säureadditionssalze und Metallkomplexe, eine bessere fungizide Wirkung besitzen als bekannte Azolverbindungen.

Die Verbindungen der Formel I enthalten asymmetrische C-Atome und können daher als Enantiomere und Diastereomere auftreten. Die Gemische von Diastereomeren lassen sich bei den erfindungsgemäßen Verbindungen in üblicher Weise, beispielsweise aufgrund ihrer unterschiedlichen Löslichkeit oder durch Säulenchromatographie trennen und in reiner Form isolieren. Die Racemate lassen sich bei den erfindungsgemäßen Verbindungen nach bekannten Methoden, beispielsweise durch Salzbildung mit einer optisch aktiven Säure, Trennung der diastereomeren Salze und Freisetzung der Enantiomeren mittels einer Base trennen. Als fungizide Mittel kann man sowohl die einheitlichen Diastereomere bzw. Enantiomere als auch deren bei der Synthese anfallenden Gemische verwenden.

$R^1$ und $R^2$ bedeuten $C_1$-$C_8$-Alkyl, insbesondere $C_1$-$C_4$-Alkyl, (Methyl, Ethyl, Isopropyl, n-Propyl, n-Butyl, sec-Butyl, tert.-Butyl, n-Pentyl, Neopentyl), 1-Naphthyl, 2-Naphthyl, p-Biphenyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, $C_1$-$C_4$-Alkoxyphenyl, 2-Methoxyphenyl, 3-Methoxyphenyl, 4-Methoxyphenyl, 2,4-Dimethoxyphenyl, 4-Ethylphenyl, 4-Isopropylphenyl, 4-tert.-Butylphenyl, $C_1$-$C_4$-Alkylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, Cyclopentyl, Cyclohexyl, 2-Cyclohexenyl, 3-Cyclohexenyl.

Säureadditionssalze sind beispielsweise die Hydrochloride, Bromide, Sulfate, Nitrate, Phosphate, Oxalate, oder Dodecylbenzolsulfonate. Die Wirksamkeit der Salze geht auf das Kation zurück, so daß es auf das Anion i.a. nicht ankommt. Die erfindungsgemäßen Wirkstoffsalze werden hergestellt durch Umsetzung der Azolylmethyloxirane (I) mit den Säuren.

Metallkomplexe der Wirkstoffe I oder ihrer Salze können z.B. mit Kupfer, Zink, Zinn, Mangan, Eisen, Kobalt oder Nickel gebildet werden, indem man die Azolylmethyloxirane mit entsprechenden Metallsalzen umsetzt, z.B. mit Kupfersulfat, Zinkchlorid, Zinnchlorid, Mangansulfat.

Die Verbindungen der Formel I können z.B. hergestellt werden, indem man eine Verbindung der Formel II

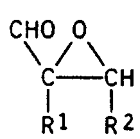

II

in welcher $R^1$ und $R^2$ die angegebenen Bedeutungen haben, mit einer Verbindung der Formel III

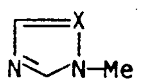

III

in der Me ein Wasserstoffatom oder ein Metallatom (Na, K) bedeutet und X die oben angegebene Bedeutung hat haben, in Gegenwart von Thionylchlorid zur Umsetzung bringt.

Die Umsetzung erfolgt z.B. gegebenenfalls in Gegenwart eines Lösungs- oder Verdünnungsmittels bei Temperaturen zwischen -30 und 80 °C. Zu den bevorzugten Lösungs- und Verdünnungsmitteln gehören Nitrile wie Acetonitril oder Propionitril, Ether wie Tetrahydrofuran, Diethylether, Dimethoxyethan, Dioxan oder Diisopropylether und insbesondere Kohlenwasserstoffe und Chlorkohlenwasserstoffe wie Pentan, Hexan, Toluol, Methylenchlorid, Chloroform, Tetrachlorkohlenstoff, Dichlorethan oder entsprechende Gemische.

Die neuen Ausgangsverbindungen II erhält man z.B. durch Epoxidierung der entsprechenden Olefine IV

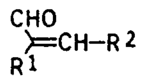

IV

in denen $R^1$ und $R^2$ die oben angegebenen Bedeutungen haben, mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, chloroform, Tetrachlorkohlenstoff, Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat, Triton B. Man arbeitet z.B. zwischen 10 und 100 °C und katalysiert die Reaktion gegebenenfalls z.B. mit Jod, Natriumwolframat oder Licht. Zur Oxidation eignen sich auch alkalische Lösungen von Wasserstoffperosid (ca. 30%ig) in Methanol, Ethanol, Aceton oder Acetonitril bei 25 bis 30 °C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Die Verbindungen IV lassen sich entsprechend allgemein bekannten Verfahren zur Aldehydsynthese (Houben-Weyl-Müller, Methoden der Organischen Chemie, Georg Thieme Verlag, Stuttgart 1983, Bd E 3) herstellen.

Die folgenden Beispiele erläutern die Herstellung der Wirkstoffe.

1. Herstellung der Ausgangsstoffe

Vorschrift 1
E/Z-2-(4-Fluorphenyl)-3-(2-chlorphenyl)-propenal

Zu einer Lösung von 35 g 2-Chlorbenzaldehyd in 300 ml Methanol werden 4,2 g Natriumhydroxid in 30 ml Wasser gegeben. Das Reaktionsgemisch wird auf 10 °C gekühlt und schnell 36 g 4-Fluorphenylacetaldehyd zugesetzt, wobei die Temperatur in der Lösung auf 30 - 40 °C ansteigt. Nach 10-stündigem Rühren bei 40 °C werden die ausgefallenen Kristalle aus der abgekühlten Reaktionslösung abgesaugt.

Vorschrift 2
cis-2-Formyl-2-(4-fluorphenyl)-3-(2-chlorphenyl)-oxiran

78,2 g E-2-(4-Fluorphenyl)-3-(2-chlorphenyl)-propenal werden in 300 ml Methanol gelöst und 1 ml Natronlauge (konz.) zugesetzt. Die Reaktionslösung wird bei 0 °C gerührt, während 20,5 g Wasserstoffperoxyd (ca. 50 %ig) langsam zugetropft werden, wobei die Innentemperatur von 30 °C nicht überschritten wird. Nach beendeter Zugabe wird sechs Stunden bei (20 °C) Raumtemperatur gerührt, anschließend 100 ml Wasser zugesetzt und die entstandene Emulsion mit Methyl-tert.-butylether ausgeschüttelt. Die isolierte organische Phase wird daraufhin über Natriumsulfat getrocknet und eingeengt. Man erhält 52,5 g (63 %) cis-2-Formyl-2-(4-fluorphenyl)-3-(2-chlorphenyl)-oxiran.

Herstellung der Endprodukte

Beispiel 1

Zu einer Lösung von 29,7 g Triazol in 150 ml Methylenchlorid wird bei 0 °C unter Stickstoffatmosphäre 12,8 g Thionylchlorid zugesetzt. Nach beendeter Zugabe wird bei Raumtemperatur für 30 Minuten gerührt und anschließend 20 g cis-2-Formyl-2-(4-fluorphenyl)-3-(2-chlorphenyl)-oxiran zugegeben. Nachdem das Reaktionsgemisch 12 - 15 Stunden bei Raumtemperatur rührte, wird der Lösung 100 ml Wasser zugesetzt und die organische Phase abgetrennt. Die verbleibende wässrige Phase wird zweimal mit Methylenchlorid ausgeschüttelt und die gesammelten organischen Phasen zweimal mit gesättigter Natriumhydrogencarbonat-Lösung gewaschen. Die isolierte organische Phase wird daraufhin über Natriumsulfat getrocknet und eingeengt, wobei 23,7 g (85 %) 1'RS-cis-2-[1-(1,2,4-Triazol-1-yl)-1-chlor-methyl]-2-(4-fluorphenyl)-3-(2-chlorphenyl)-oxiran als 2:1-Diastereomerengemisch erhalten werden. Man erhält aus Methyl-tert.-butylether 5,8 g des vermehrt gebildeten Diastereomeren A mit dem Schmelzpunkt 152-156 °C (Verbindung Nr. 1).

Entsprechend Beispiel 1 können die in der Tabelle 1 aufgeführten Verbindungen hergestellt werden.

Tabelle 1

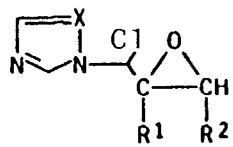

| Bsp. | $R^1$ | $R^2$ | X | Schmp/IR | Bemerkung |
|---|---|---|---|---|---|
| 1 | $4-F-C_6H_4$ | $2-Cl-C_6H_4$ | N | 152-156°C | Enantiomerengemisch |
| 2 | $4-F-C_6H_4$ | $2-Cl-C_6H_4$ | CH | 144-152°C | 1:1-Diastereomerengemisch |
| | | | | 151-155°C | Enantiomerengemisch |
| 3 | $4-F-C_6H_4$ | $4-Cl-C_6H_4$ | N | | |
| 4 | $4-F-C_6H_4$ | $4-Cl-C_6H_4$ | CH | | |
| 12 | $4-F-C_6H_4$ | $2,4-Cl_2-C_6H_3$ | N | | |
| 5 | $4-F-C_6H_4$ | $2-Cl-4-F-C_6H_3$ | N | | |
| 6 | $4-F-C_6H_4$ | $2-CF_3-C_6H_4$ | N | 126-127°C | Enantiomerengemisch |
| 7 | $4-F-C_6H_4$ | $4-CF_3-C_6H_4$ | N | | |

Tabelle 1 (Fortsetzung)

| Bsp. | $R^1$ | $R^2$ | X | Schmp/IR | Bemerkung |
|---|---|---|---|---|---|
| 8 | $4-F-C_6H_4$ | $2-F-C_6H_4$ | N | | |
| 9 | $4-F-C_6H_4$ | $4-F-C_6H_4$ | N | | |
| 10 | $4-F-C_6H_4$ | $2,4-F_2-C_6H_3$ | N | Harz | 1:1-Diastereomerengemisch |
| 11 | $4-F-C_6H_4$ | $2,4-F_2-C_6H_3$ | CH | 118-123°C | 1:1-Diastereomerengemisch |
| 12 | $4-F-C_6H_4$ | $2-OCH_3-C_6H_4$ | N | 118-124°C | Enantiomerengemisch |
| 13 | $4-F-C_6H_4$ | 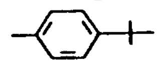 | N | | |
| 14 | $4-F-C_6H_4$ | $4-NO_2-C_6H_4$ | N | | |
| 15 | $4-F-C_6H_4$ | $4-NH_2-C_6H_4$ | N | | |
| 16 | $4-F-C_6H_4$ | $2-C_{10}H_7$ | N | | |
| 17 | $4-F-C_6H_4$ | Cyclopropyl | N | | |
| 18 | $4-F-C_6H_4$ | Cyclohexyl | N | | |
| 19 | $C_6H_5$ | $2-Cl-C_6H_4$ | N | | |
| 20 | $C_6H_5$ | $2-Cl-C_6H_4$ | CH | | |
| 21 | $C_6H_5$ | $4-Cl-C_6H_4$ | N | 136-138°C | Enantiomerengemisch |
| 22 | $C_6H_5$ | $4-Cl-C_6H_4$ | CH | 167-172 | E:1-Diastereomerengemisch |

Tabelle 1 (Fortsetzung)

| Bsp. | $R^1$ | $R^2$ | X | Schmp/IR | Bemerkung |
|---|---|---|---|---|---|
| 23 | $C_6H_5$ | $2,4-Cl_2-C_6H_3$ | N | | |
| 24 | $C_6H_5$ | $2-F-C_6H_4$ | N | | |
| 25 | $C_6H_5$ | $4-F-C_6H_4$ | N | | |
| 26 | $C_6H_5$ | $4-CF_3-C_6H_4$ | N | | |
| 27 | $C_6H_5$ | $2-OCH_3-C_6H_4$ | N | | |
| 28 | $C_6H_5$ | Cyclohexyl | N | | |
| 29 | $2-Cl-C_6H_4$ | $2-Cl-C_6H_4$ | N | | |
| 30 | $2-Cl-C_6H_4$ | $4-Cl-C_6H_4$ | N | | |
| 31 | $4-Cl-C_6H_4$ | $2-Cl-C_6H_4$ | N | | |
| 32 | $4-Cl-C_6H_4$ | $4-Cl-C_6H_4$ | N | | |
| 33 | $4-Cl-C_6H_4$ | $2-F-C_6H_4$ | N | | |
| 34 | $4-Cl-C_6H_4$ | $4-F-C_6H_4$ | N | | |
| 35 | $4-Cl-C_6H_4$ | $2-CF_3-C_6H_4$ | N | | |
| 36 | $2,4-Cl_2-C_6H_3$ | $2-Cl-C_6H_4$ | N | | |
| 37 | $2,4-Cl_2-C_6H_3$ | $2,4-Cl_2-C_6H_3$ | N | | |
| 38 | $4-Br-C_6H_4$ | $2-Cl-C_6H_4$ | N | | |
| 39 | Cyclohexyl | $2-Cl-C_6H_4$ | N | | |
| 40 | Cyclohexyl | $4-Cl-C_6H_4$ | N | | |
| 41 | Cyclohexyl | $2-F-C_6H_4$ | N | | |
| 42 | Cyclohexyl | $4-F-C_6H_4$ | N | | |
| 43 | Cyclohexyl | $4-F-C_6H_4$ | CH | | |

Tabelle 1 (Fortsetzung)

| Bsp. | $R^1$ | $R^2$ | X | Schmp/IR | Bemerkung |
|---|---|---|---|---|---|
| 44 | Cyclohexyl | $2,4-Cl_2-C_6H_3$ | N | | |
| 45 | Cyclohexyl | Cyclohexyl | N | | |
| 46 | $CH_3$ | $2-Cl-C_6H_4$ | N | | |
| 47 | $CH_3$ | $4-F-C_6H_4$ | N | | |
| 48 | $4-F-C_6H_9$ | $3-F-C_6H_4$ | N | Harz | 2:1 Diastereomerengemisch |
| 49 | $4-F-C_6H_4$ | $4-CF_3-C_6H_4$ | CH | 90-94°C | 2:1-Diastereomerengemisch |
| 50 | $4-F-C_6H_4$ | $2-Br-C_6H_4$ | N | 151-158°C | Enantiomerengemisch |
| 51 | $4-F-C_6H_4$ | $2-Br-C_6H_4$ | CH | 159-164°C | Enantiomerengemisch |
| 52 | $4-F-C_6H_4$ | $C_6H_5$ | N | 134-138°C | 12:1-Diastereomerengemisch |
| 53 | $4-F-C_6H_4$ | $C_6H_5$ | CH | Harz | 1:1-Diastereomerengemisch |
| 54 | $C_6H_5$ | $2,4-Cl_2-C_6H_3$ | CH | 176-180°C | Enantiomerengemisch |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 21 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 22 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 21 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 22 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 21 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 22 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 21 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 22 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 21 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiel

Als Vergleichswirkstoffe wurden 2-(1,2,4-Triazol-1-yl-methyl)-2-phenyl-3-(4-chlorphenyl)-oxiran (A) und 2-(Imidazol-1-yl-methyl)-2-phenyl-3-(4-chlorphenyl)-oxiran (B) - bekannt aus DE 3 218 130 - benutzt.

Wirksamkeit gegen Weizenmehltau

Blätter von in Töpfen gewachsenen Weizenkeimlingen der Sorte "Kanzler" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22 °C und 75 bis 80 % relativer Luftfeuchtigkeit aufgestellt. Nach 7 Tagen wurde das Ausmaß der Mehltauentwicklung ermittelt.

Das Ergebnis zeigt, daß die Wirkstoffe 21 und 22 bei der Anwendung als 0,025 %ige (Gew.%) Spritzbrühe eine bessere fungizide Wirkung zeigen (95 %) als die bekannten Vergleichswirkstoffe A und B (80 %).

## Patentansprüche

**1.** 1-Halogen-1-azolylmethyloxirane der Formel I

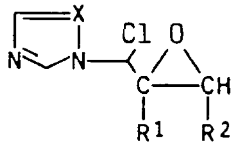

I

in welcher
$R^1$ und $R^2$ gleich oder verschieden sind und $C_1$-$C_8$-Alkyl, $C_5$-$C_8$-Cycloalkyl, $C_5$-$C_8$-Cycloalkenyl, Naphthyl, Biphenyl oder Phenyl bedeuten, wobei diese Reste einfach bis dreifach durch Halogen, Nitro,

Phenoxy, Amino, Alkyl, Alkoxy oder Halogenalkyl mit jeweils 1 bis 4-C-Atomen substituiert sein können, X den Rest CH oder N bedeutet, sowie deren für Pflanzen verträgliche Säureadditionssalze und Metallkomplexe.

**2.** 1-Halogen-1-azolylmethyloxirane der allgemeinen Formel I, in denen $R^1$ und $R^2$ die Phenylgruppe bedeutet, welche unsubstituiert oder durch einen oder zwei Substituenten substituiert ist, die Fluor, Chlor, Brom oder die Trifluormethylgruppe bedeuten.

**3.** Verfahren zur Herstellung der 1-Halogen-1-azolylmethyloxirane der Formel I gemäß Anspruch 1, dadurch gekennzeichnet, daß man eine Verbindung der Formel II

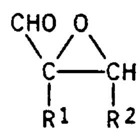

II

in welcher $R^1$ und $R^2$ die angegebenen Bedeutungen haben, mit einer Verbindung der Formel III

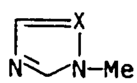

III

in der Me ein Wasserstoffatom oder ein Metallatom (Na, K) bedeutet und X die oben angegebene Bedeutung hat, in Gegenwart von Thionylhalogeniden umsetzt.

**4.** Fungizides Mittel, enthaltend einen Trägerstoff und ein 1-Halogen-1-azolylmethyloxiran der Formel I gemäß Anspruch 1.

**5.** Verfahren zur Bekämpfung von Pilzen, dadurch gekennzeichnet, daß man eine fungizid wirksame Menge eines 1-Halogen-1-azolylmethyloxirans der Formel I gemäß Anspruch 1 auf die Pilze oder auf durch Pilzbefall bedrohte Materialien, Pflanzen, Flächen oder Saatgüter einwirken läßt.

**6.** Verbindung der Formel I gemäß Anspruch 1, dadurch gekennzeichnet, daß $R^1$ Phenyl, $R^2$ 4-Chlorphenyl und X den Rest N bedeutet.

**7.** Verbindung der Formel I gemäß Anspruch 1, dadurch gekennzeichnet, daß $R^1$ Phenyl, $R^2$ 4-Chlorphenyl und X den Rest CH bedeutet.

## Claims

**1.** A 1-halo-1-azolylmethyloxirane of the formula I

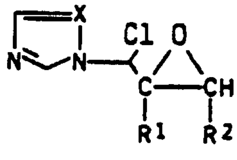

I

where $R^1$ and $R^2$ are identical or different and are each $C_1$-$C_8$-alkyl, $C_5$-$C_8$-cycloalkyl, $C_5$-$C_8$-cycloalkenyl, naphthyl, biphenyl or phenyl, which radicals may be monosubstituted to trisubstituted by halogen, nitro, phenoxy, amino, alkyl, alkoxy or haloalkyl, each of 1 to 4 carbon atoms, and X is CH or N, or a plant-tolerated acid addition salt or metal complex thereof.

**2.** A 1-halo-1-azolylmethyloxirane of the general formula I where $R^1$ and $R^2$ are each phenyl which is unsubstituted or substituted by one or two substituents selected from the group consisting of fluorine,

chlorine, bromine and trifluoromethyl.

3. A process for the preparation of a 1-halo-1-azolylmethyloxirane of the formula I as claimed in claim 1, which comprises reacting a compound of the formula II

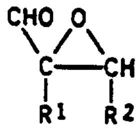

II

where $R^1$ and $R^2$ have the stated meanings, with a compound of the formula III

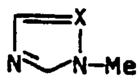

III

where Me is hydrogen or a metal atom (Na, K) and X has the abovementioned meaning in the presence of a thionyl halide.

4. A fungicidal composition comprising a carrier and a 1-halo-1-azolylmethyloxirane of the formula I as claimed in claim 1.

5. A method of controlling fungi, which comprises applying a fungicidally effective amount of a 1-halo-1-azolylmethyloxirane of the formula I as claimed in claim 1 to the fungi or to materials, plants, areas or seed threatened by fungal attack.

6. A compound of the formula I as claimed in claim 1, wherein $R^1$ is phenyl, $R^2$ is 4-chlorophenyl and X is N.

7. A compound of the formula I as claimed in claim 1, wherein $R^1$ is phenyl, $R^2$ is 4-chlorophenyl and X is CH.

## Revendications

1. 1-halogène-1-azolylméthyloxirranes de formule I

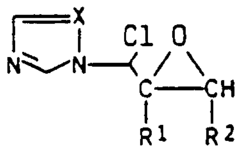

I

dans laquelle $R^1$ et $R^2$ sont identiques ou différents et représentent alkyle en $C_1$-$C_8$, cycloalkyle en $C_5$-$C_8$, cycloalcényle en $C_5$-$C_8$, naphtyle, biphényle ou phényle, ces restes pouvant être substitués une à trois fois par halogène, nitro, phénoxy, amino, alkyle, alcoxy ou halogénoalkyle de chacun 1 à 4 atomes C.
X représente le reste CH ou N
ainsi que leurs sels d'addition d'acide et complexes métalliques acceptables pour les plantes.

2. 1-halogène-1-azolylméthyloxirranes de la formule I, dans laquelle $R^1$ et $R^2$ représentent le groupe phényle, qui est non substitué ou substitué par un ou deux substituants qui représentent fluor, chlore, brome ou le groupe trifluorométhyle.

3. Procédé de préparation de 1-halogène-1-azolylméthyloxirranes de formule I selon la revendication 1, caractérisé par le fait que l'on fait réagir, en présence d'halogénures de thionyle, un composé de

formule II

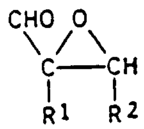

II

dans laquelle $R^1$ et $R^2$ ont les significations indiquées, avec un composé de formule III

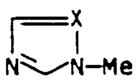

III

dans laquelle Me représente un atome d'hydrogène ou un atome métallique (Na, K) et X a la signification sus-indiquée.

**4.** Fongicide contenant un support et un 1-halogène-1-azolylméthyloxirrane de formule I selon la revendication 1.

**5.** Procédé de lutte contre les champignons, caractérisés par le fait que l'on fait réagir une quantité efficace du point de vue fongicide d'un 1-halogène-1-azolylméthyloxirrane de formule I selon la revendication 1 sur les champignons ou sur les matériaux, plantes, surfaces ou semences menacées par une attaque par champignons.

**6.** Composé de formule I selon la revendication 1, caractérisé par le fait que $R^1$ représente phényle, $R^2$, chlorophényle et X le reste N.

**7.** Composé de formule I selon la revendication 1, caractérisé par le fait que $R^1$ représente phényle, $R^2$ chlorophényle et X le reste CH.